# FASCICULE DE BREVET EUROPEEN

(11) **EP 3 341 041 B1**
(45) Date de publication et mention de la délivrance du brevet: **25.11.2020**
(21) Numéro de dépôt: 16744342.3
(22) Date de dépôt: 19.07.2016
(51) Int. Cl.: A61M 1/10, A61M 1/12

(54) **POMPE DE FLUX SANGUIN POUR ASSISTANCE VENTRICULAIRE**
BLUTFLUSSPUMPE ZUR VENTRIKULÄREN UNTERSTÜTZUNG
BLOOD FLOW PUMP FOR VENTRICULAR ASSISTANCE

(30) Priorité: 25.08.2015 FR 1557893
(43) Date de publication de la demande: 04.07.2018
(73) Titulaire: Fineheart, 33600 Pessac (FR)
(72) Inventeur: HADDADI, Mohammad, 33700 Merignac (FR)
(74) Mandataire: IPAZ
(86) Numéro de dépôt international: PCT/EP2016/067121
(87) Numéro de publication internationale: WO 2017/032510

(56) Documents cités:
- EP-A2- 1 481 698
- JP-A- H07 178 163
- US-A1- 2014 341 726

## Description

La présente invention se rapporte à une pompe pour assistance ventriculaire. Il s'agit par exemple d'une pompe alimentée par batterie et destinée à être insérée dans un corps humain pour aider à la circulation du sang (voir p.ex. US2014/0341726).

Dans le domaine des systèmes d'assistance ventriculaire, on connaît deux types de pompes: la pompe axiale qui génère un débit important avec une faible augmentation de pression, et la pompe centrifuge qui génère une forte pression avec un faible débit.

Afin de savoir quel type de pompe est la plus adaptée pour une utilisation donnée, on est souvent amené à estimer une vitesse spécifique du sang dans la pompe. La vitesse spécifique est une variable réduite calculée en fonction de différents paramètres: le débit désiré, la hauteur d'élévation (différence de pression entre l'entrée et la sortie) et la vitesse de rotation. A partir de cette valeur, le choix de la pompe peut être fait parmi les modes suivants : centrifuge pour une vitesse comprise dans [0-1,2], mixte pour une vitesse spécifique comprise dans [1-2,2] et axiale au-dessus. Des abaques permettent de choisir un type de pompe avec un profil prédéfini pour les ailettes ou pales utilisées dans le rotor.

Quel que soit le type de pompe choisi, on constate la présence de forces de cisaillement dans la plupart des pompes de l'art antérieur du fait que les rotors et carter utilisées dans l'art antérieur créent des tourbillons excessifs dans le sang. Les forces de cisaillement sont à l'origine de la création d'hémolyse, c'est-à-dire la destruction de globules rouges. Lorsque les globules rouges sont détruits, quel que soit le débit pour véhiculer le sang, l'oxygène ne parvient pas aux cellules.

Un autre inconvénient est la stagnation de sang provoquant des caillots de sang, à l'origine de la thrombose.

La présente invention a pour objet une pompe évitant la création de thrombose.

Un autre objet de l'invention et d'éviter la création d'hémolyse.

On atteint au moins l'un des objectifs avec une pompe destinée à être immergée dans un fluide, cette pompe comprenant :
- un inducteur doté de pales de guidage pour rendre l'écoulement du fluide linéaire ;
- un rotor comprenant un corps central de forme évasée, ce rotor étant destiné à créer de l'énergie cinétique et étant disposé en aval de l'inducteur par rapport au sens de circulation du fluide ;
- un arbre (17) d'entrainement et de maintien du rotor (5,16), cet arbre étant fixé sur l'extrémité amont du rotor (5.16) et traversant la chambre d'admission (3) dans sa zone axiale ;
- au moins une pale hélicoïdale réalisée autour dudit corps central ; cette pale hélicoïdale ayant un profil extérieur évasé et comporte des spires à pas d'enroulement croissant et tendant vers l'infini, et le volume intérieur du carter étant complémentaire à la forme évasée de ladite au moins une pale hélicoïdale,
- un carter autour du rotor ;
- un diffuseur doté de pales pour rendre l'écoulement de fluide linéaire et augmenter la pression du fluide ; ce diffuseur étant disposé en aval du rotor de façon à évacuer le fluide depuis le rotor vers l'extérieur en transformant l'énergie cinétique créée par le rotor en énergie potentielle ; et
- un redresseur doté de pales et d'un orifice en sortie de diamètre inférieur au diamètre d'entrée du redresseur, les pales dirigeant le fluide, en provenance du diffuseur, vers l'orifice de façon à augmenter la vitesse et donner au fluide un profil prédéterminé en sortie de l'orifice.

En d'autres termes, la pale hélicoïdale est enroulée autour du rotor de telle sorte que l'angle d'attaque de la pale hélicoïdale diminue au fur et à mesure que l'on se déplace de l'extrémité amont du rotor vers l'extrémité en aval; l'angle d'attaque étant défini comme l'angle entre l'axe de rotation du rotor et un vecteur tangent à la surface externe de la pale hélicoïdale. On notera que la pale peut être enroulée autour du rotor comme décrit principalement dans la présente demande, mais elle peut également être positionnée sur la paroi interne du carter.

L'écoulement de fluide linéaire telle que défini ici, l'est par opposition à un écoulement tourbillonnant. Cet écoulement linéaire peut être laminaire.

Le redresseur (« straightener » en langue anglaise) selon l'invention permet de créer un flux en concentrant le fluide de façon à obtenir des valeurs de vitesses élevées en sortie de la pompe. De façon générale, le système vasculaire d'un cœur présente des résistances vasculaires assez élevées. Une pompe efficace est une pompe capable de propulser le sang dans les valves avec une pression suffisante pour vaincre ces résistances vasculaires. La pression en sortie de la pompe est primordiale comparativement à la vitesse de sortie qui avec la pompe selon l'invention peut atteindre la vitesse maximale de 3 m/s.

En d'autres termes, le redresseur permet de canaliser le fluide, de créer un profil permettant d'avoir une vitesse maximale en un point, *i.e.* aligné avec la valve aortique, de manière à expulser le flux de façon laminaire. Ceci permet d'éviter la création de tourbillons en sortie de la pompe. Le diamètre de l'orifice de sortie est plus petit que le diamètre interne du carter. Son petit diamètre de sortie, par exemple un demi ou un tiers du diamètre interne du carter, permet de régler les paramètres de pression, entre 80 et 200 mmHg, et de vitesse, entre 1 et 3 m/s, tout en évitant la création d'un courant à vitesse négative (en contresens par manque de pression et d'homogénéité du flux en sortie).

L'ensemble diffuseur et redresseur selon l'invention permet donc de rendre la pompe efficace. Le redresseur diffuse le fluide directement vers la sortie dans le milieu ambiant en créant un flux. Ce milieu ambiant peut avantageusement être ledit fluide, qui est de préférence du sang.

L'inducteur selon l'invention évite le phénomène de cavitation, c'est-à-dire la création de bulle dans le fluide.

Il est important de noter que cette pompe est parfaitement adaptée pour un fonctionnement selon une disposition verticale, ou légèrement penchée c'est-à-dire inclinée entre 0 et 5 degrés par rapport à l'axe verticale. La pompe selon l'invention peut également fonctionner couchée comme la plupart des pompes de l'art antérieur.

Concernant la pale hélicoïdale, son profil extérieur est évasé et le volume intérieur du carter est complémentaire à cette forme évasée. Dans la plupart des pompes de l'art antérieur, le carter présente un volume intérieur cylindrique droit de sorte que le profil extérieur des pales est plutôt rectangulaire. Il s'agit notamment de pompes axiales où le diamètre extérieur des pales est identique au diamètre intérieur du carter de profil rectangulaire, ce sont des pompes isodiamétriques. Une telle conception de profil rectangulaire n'est pas efficace lorsque la pompe doit être disposée à la verticale.

La pompe selon l'invention peut en outre comprendre une chambre d'admission pourvue d'ouvertures latérales pour que le fluide puisse pénétrer radialement puis s'engager dans l'axe vers l'inducteur. Cette chambre d'admission peut être de forme cylindrique comportant sur sa partie supérieure en aval desdites ouvertures, un réceptacle pour accueillir l'inducteur. La chambre d'admission et l'inducteur peuvent constituer deux pièces destinées à être fermement solidaires l'une de l'autre ou bien être conçus comme une seule et unique pièce.

Les pales de guidage de l'inducteur sont avantageusement conçues de façon à laisser passer une tige permettant de relier le rotor à un moteur, l'inducteur se trouvant entre le carter et ce moteur. En fonctionnement, le corps central (avec la pale hélicoïdale) est en rotation à l'intérieur du carter sans entrer en contact avec les autres composants de la pompe.

Selon un mode de réalisation nullement limitatif, la pompe comporte en tout quatre pales hélicoïdales identiques réparties de façon homogène autour du corps central.

De préférence, le corps central du rotor est de forme oblongue, c'est-à-dire que les flancs du corps central peuvent présenter des courbures. La tête de ce corps central peut être arrondie et dépourvue de pale, par exemple sous forme d'ogive.

Selon l'invention, le diffuseur peut être un cylindre creux doté de pales de guidage droites réparties dans sa paroi interne et s'étendant de la périphérie vers le centre. Son rôle est de transformer une partie de l'énergie cinétique du fluide en pression, cette pression étant notamment transférée au redresseur. En complément notamment, les pales de guidage du diffuseur peuvent présenter une forme vrillée dans un sens inverse au sens d'enroulement de la pale hélicoïdale autour du corps central.

Selon une caractéristique avantageuse de l'invention, lorsque le corps central comporte une tête arrondie dépourvue de pales, le diffuseur coiffe cette tête arrondie, et chaque pale de guidage du diffuseur présente une forme complémentaire à une partie de la tête arrondie faisant face. Le diamètre interne du cylindre formant le diffuseur est identique au diamètre interne du carter au niveau de l'extrémité aval de la pale hélicoïdale.

Selon l'invention, le redresseur est disposé en aval du diffuseur selon le sens de circulation du fluide. Par exemple, la paroi interne du redresseur peut être de forme conique avec, disposées dessus, des pales de guidage droites.

Selon une variante du diffuseur selon l'invention, ce diffuseur peut comporter une partie centrale en forme d'ogive pointue vers l'aval, un cylindre autour de la base de l'ogive, des pales de guidage reliant ce cylindre à la base de l'ogive ; ce diffuseur étant destiné à venir en prise avec l'extrémité aval du rotor via un roulement permettant de garder le diffuseur fixe par rapport au rotor. Un tel diffuseur permet d'améliorer la directivité, la pression et l'homogénéité du flux en sortie de la pompe.

De la même manière, dans ce mode de réalisation les pales de guidage du diffuseur peuvent également être vrillées dans un sens inverse au sens d'enroulement de la pale hélicoïdale autour du corps central. Ainsi le diffuseur fait opposition au caractère tourbillonnant du fluide arrivant du rotor. Après le passage du fluide dans le diffuseur, celui-ci est complètement corrigé de façon à constituer un flux laminaire par l'action du redresseur.

En particulier, selon la variante, le redresseur est disposé en aval du diffuseur selon le sens de circulation du fluide ; des pales de guidage sont droites et sont conçues de façon à laisser pénétrer la tête de l'ogive dans la partie centrale du redresseur.

D'une façon générale, l'ensemble rotor et pale hélicoïdale peut avantageusement présenter un profil de type centrifuge du côté amont, de type mixte dans la partie centrale, et de type axial du côté aval.

Avec la pompe selon l'invention, l'ensemble rotor et carter présente les caractéristiques à la fois :
- d'une pompe de type centrifuge, c'est-à-dire une accélération radiale du fluide arrivant axialement, pour ce faire la partie basse des pales hélicoïdales présente un angle prononcé, de l'ordre de l'ordre de 45 degrés, par exemple entre 40 et 50 degrés, par rapport à l'axe de rotation du rotor,
- d'une pompe de type mixte, c'est-à-dire une pente ou une courbure moins prononcée des spires des pales hélicoïdales, et
- d'une pompe de type axial, le fluide étant conduit selon une direction parallèle à l'axe de rotation du rotor.

Cette configuration permet de limiter les forces de cisaillement (« shear stress » en langue anglaise) qui peuvent engendrer une hémolyse, c'est-à-dire une destruction des globules rouges. Même avec un fort débit, lorsque les globules rouges sont détruits, l'oxygène ne parvient pas jusqu'aux cellules.

Les forces de cisaillement sont créées par des effets de tourbillonnement de sang en entrée et en sortie de pompe. La pompe selon l'invention évite les tourbillons par un appel de sang en utilisant une structure de type centrifuge et un refoulement axial.

Selon une caractéristique avantageuse de l'invention, la partie du côté amont de l'ensemble corps central et pale hélicoïdale est dimensionnée pour une vitesse spécifique comprise entre 0 et 1.2. Par ailleurs, la partie centrale de l'ensemble corps central et pale hélicoïdale peut être dimensionnée pour une vitesse spécifique comprise entre 1 et 2.2. Enfin, la partie centrale de l'ensemble corps central et pale hélicoïdale peut être dimensionnée pour une vitesse spécifique supérieure à 2.2. Il s'agit de valeur de vitesse spécifique permettant de classifier les structures centrifuge, mixte ou axial respectivement.

Lors de l'étude théorique préliminaire avant conception, en fonction de son utilisation et des objectifs en termes de débit à atteindre, il a été constaté que la pompe selon l'invention devrait avoir une vitesse spécifique de l'ordre de 1. Les abaques dans le domaine technique de l'invention et les préjugés communément admis auraient poussés naturellement l'homme du métier vers un choix de pompe ou turbine de type centrifuge ou axial. Cependant, la pompe selon l'invention présente un dessin très particulier et innovant où les trois types de pompe sont présents, y compris le type mixte. La structure de la pompe selon l'invention ne suit pas la méthode traditionnelle (« handbook ») de dessin d'une pompe ou turbine de type homogène.

Selon une caractéristique de l'invention, la pompe comporte un arbre d'entrainement et de maintien du rotor, cet arbre étant fixé sur l'extrémité amont du rotor et traversant la chambre d'admission dans sa zone axiale. Cet arbre est notamment relié à un moteur apte à mettre en mouvement le rotor.

Selon l'invention, la chambre d'admission, l'inducteur le carter et le diffuseur sont conçus en une seule pièce ou sont fermement fixés les uns aux autres sans mouvements relatifs, le rotor étant maintenu mobile en rotation dans le carter.

L'inducteur dans la chambre d'admission, le diffuseur et le redresseur sont également des éléments fixes par rapport au rotor.

De préférence, la pale hélicoïdale présente une hauteur radiale (son épaisseur dans la direction radiale) identique sur toute sa longueur (de bas en haut du corps central). En variante, on peut envisager de préférence une hauteur radiale (son épaisseur dans la direction radiale) variable sur toute sa longueur. On peut prévoir une hauteur de 1 à 4 mm en bas et 1 à 3 mm en haut du corps central, avec une progression continue ou par paliers, en fonction des caractéristiques de pression et de vitesse dont on a besoin.

La forme globale extérieure de la pompe est un cylindre de section circulaire, mais d'autres types de sections peuvent être envisagées telles qu'une section carrée, triangulaire ; la chambre d'admission peut présenter une forme globale différente de la forme globale du carter.

Selon un autre aspect de l'invention, il est prévu un procédé de pilotage de la pompe telle que définie ci-dessus. Dans ce procédé, le rotor est actionné par un moteur, connecté à la pompe, conformément au rythme cardiaque. Le sang est pulsé hors de la pompe selon le rythme de la pulsation cardiaque, notamment de type sinusoïdal avec des accélérations et des ralentissements. Le rythme cardiaque peut être détecté au moyen d'une sonde reliant le cœur à une unité de commande qui contrôle le moteur.

La pompe s'adapte en rotation : si le cœur bat à 60 pulsations ou à 120 pulsations par minute, la pompe selon l'invention suit le rythme alors que la plupart des pompes de l'art antérieur fonctionne à régime fixe.

D'autres avantages et caractéristiques de l'invention apparaîtront à l'examen de la description détaillée d'un mode de mise en œuvre nullement limitatif, et des dessins annexés, sur lesquels :
La figure 1 est une vue générale extérieure de la pompe selon l'invention,
La figure 2 est une vue intérieure par transparence de la pompe selon l'invention,
La figure 3 est une vue éclatée de la pompe selon l'invention,
La figure 4 est une vue schématique en coupe longitudinale de la pompe selon l'invention,
La figure 5 est une vue en perspective de la chambre d'admission selon l'invention,
La figure 6 est une vue en perspective de l'inducteur à insérer dans la chambre d'admission selon l'invention,
La figure 7 est un schéma illustrant différentes vues du carter selon l'invention,
La figure 8 est une vue en perspective du rotor selon l'invention,
La figure 9 est une vue en perspective du diffuseur selon l'invention,
La figure 10 est une vue en perspective du redresseur selon l'invention,
La figure 11a, 11b et 11c sont des vues schématiques d'une variante de la pompe selon l'invention,
Les figures 12 et 13 sont des vues schématiques en perspective de l'avant et de l'arrière du diffuseur selon la variante des figures 11a-c,
La figure 14 est une vue schématique d'un rotor selon une variante où la tête du rotor est en forme d'ogive,
La figure 15 est une vue schématique d'un rotor selon la variante des figures 11a-c,
Les figures 16 et 17 sont des vues schématiques d'un inducteur selon l'invention,
Les figures 18 et 19 sont des vues schématiques d'un redresseur selon l'invention, et
La figure 20 est une vue en coupe schématique d'une pale avec une illustration de différents paramètres.

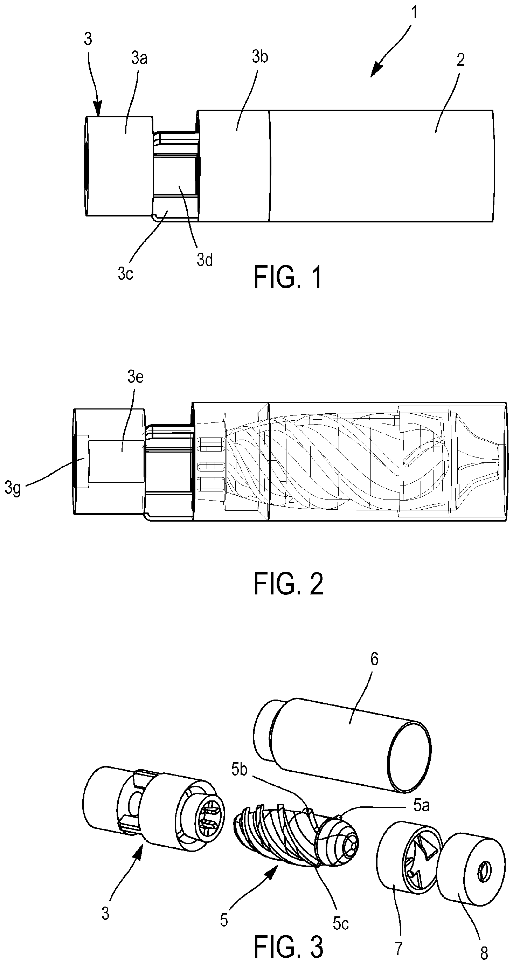

Sur la figure 1, on distingue la pompe 1 selon l'invention sous la forme globale d'un cylindre à section circulaire, destinée à aspirer un fluide tel que le sang et le refouler de façon à favoriser la circulation sanguine. Une telle pompe est destinée à être installée dans un corps notamment pour assistance ventriculaire. Sa longueur est d'environ 61.8mm, le diamètre du carter 2 est d'environ 17 - 20 mm, alors que la partie basse 3a présente un diamètre d'environ 15 - 20 mm.

La pompe selon l'invention peut avantageusement, mais pas uniquement, être utilisée dans un positionnement vertical, c'est-à-dire le carter 2 à la verticale et au-dessus de la partie basse 3a. La plupart des pompes de l'art antérieur sont utilisées dans un mode horizontal.

Selon l'invention, la chambre d'admission 3 a pour fonction de faire entrer le fluide, notamment du sang, par les ouïes ou ouvertures 3d sous l'action d'une aspiration provenant de l'intérieur du carter 2. Le fluide est ensuite refoulé par une ouverture à l'extrémité du carter.

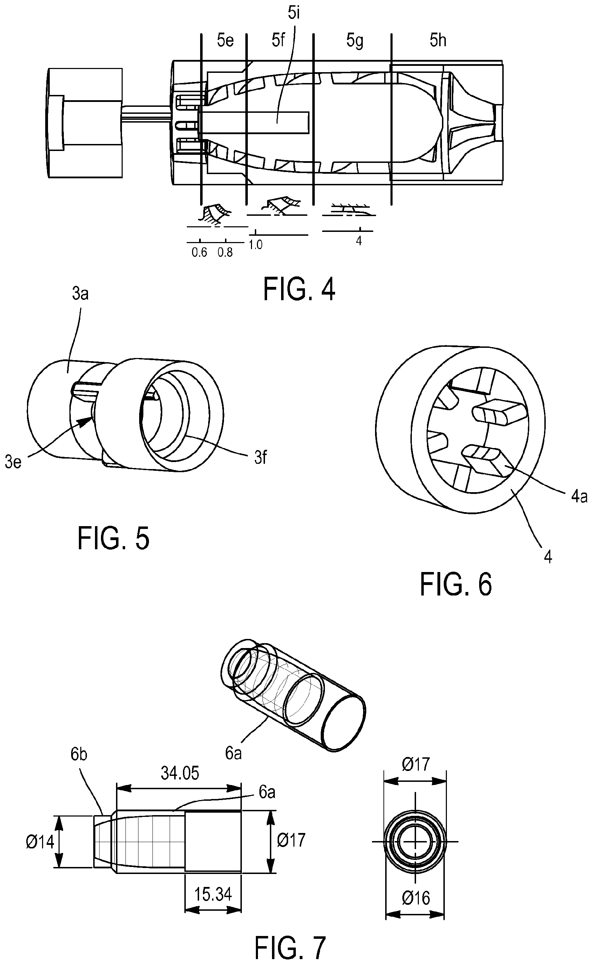

Sur la figure 5 on voit plus en détail la chambre d'admission 3 constituée d'une partie basse 3a, d'une partie supérieure 3b, les deux parties étant reliées par des guides radiaux 3c délimitant des ouvertures 3d vers l'intérieur de la chambre d'admission.

La partie basse 3a est un cylindre de section circulaire, de paroi épaisse de telle sorte que la partie centrale est un tunnel 3e. Le diamètre de ce dernier est inférieur au diamètre extérieur de la section du cylindre, qui est d'environ 15mm. Dans l'exemple de la figure 5, le diamètre du tunnel 3e est de 6 mm.

Les guides radiaux 3c sont trois plaques inscrites dans des plans qui se coupent sur l'axe de la chambre d'admission. La face extérieure de chaque plaque 3c affleure la surface extérieure latérale de la partie supérieure 3b. La zone centrale contenant l'axe de la chambre d'admission est vide pour le passage du fluide. Cette zone centrale constitue un tunnel de diamètre supérieur au diamètre du tunnel 3e.

La partie supérieure 3b est sous la forme d'un cylindre présentant deux épaisseurs différentes, une première épaisseur du côté en amont, c'est-à-dire du côté en contact avec les guides radiaux 3c, et une seconde épaisseur, inférieure à la première, du côté aval. Entre les deux épaisseurs se trouve une marche 3f. Avec une telle disposition, un inducteur 4 tel que décrit sur la figure 6 peut être inséré et fixé à l'intérieur de la chambre d'admission 3 dans la partie 3b de grande épaisseur. Lors de l'insertion, cet inducteur 4 peut venir se poser sur des extrémités des guides 3c. Les dimensions de l'inducteur 4 sont telles qu'une fois inséré, sa partie supérieure affleure la marche 3f. On peut envisager d'autres modes de réalisation telles que par exemple une pièce unique constituée des éléments 3b et 4, ou alors 3 et 4. L'inducteur 4 est un cylindre creux comportant des guides radiaux 4a, par exemple au nombre de quatre jusqu'à six, sur toute la hauteur du cylindre et s'inscrivant dans des plans radiaux concourants au centre du cylindre. L'inducteur 4 sert de guide d'entrée du fluide. Il permet de limiter la cavitation dans les étages supérieurs qui seront décrits par la suite. Les guides 4a produisent un écoulement laminaire de sorte que le caractère turbulent du fluide est considérablement réduit. Cela permet de ralentir et réduire la détérioration généralement rapide du rotor qui sera décrit ci-après, en limitant les attaques du fluide sur les pales de ce rotor.

Les différentes pièces de la pompe peuvent être conçues par moulage, impression 3D, usinage ou autres.

Sur les figures 2 et 3 on distingue en vue intérieure transparente et en vue éclatée les différentes pièces de la pompe. La vue de la figure 2 permet de distinguer un espace creux 3g de diamètre plus grand que celui du tunnel 3e.

Les étages supérieurs de la chambre d'admission comprennent un rotor 5 destiné à se mouvoir à l'intérieur d'un carter 6, et des éléments de sortie tels un diffuseur 7 et un redresseur 8. Le rotor 5 visible sur les figures 2, 3 et 4 présente une forme oblongue ou ovale avec un seul axe de symétrie : c'est-à-dire comme une ogive allongée ou étirée sur une extrémité. Le rotor 5 est donc un corps dont le diamètre de la section circulaire (coupe radiale) croît depuis sa partie basse jusque vers la partie supérieure, puis décroît rapidement jusqu'à l'extrémité supérieure. On peut définir une partie utile comme étant celle pour laquelle le diamètre croît. Cette croissance est de préférence continue mais pas linéaire de sorte que la forme extérieure de la partie utile est de type conique à paroi convexe.

Avantageusement, on réalise entre trois et cinq pales. Sur la figure 2, il y a quatre pales hélicoïdales 5a, 5b, 5c, 5d, sur la partie utile. Chaque pale hélicoïdale est un serpentin d'épaisseur constante ou variable sur toute la longueur de sorte que la forme extérieure du rotor avec les pales hélicoïdales à pas évolutif qui reste conique à paroi convexe. Cette forme innovante du rotor 5 présente une partie supérieure en demi-sphère 5h et la partie utile, cette dernière pouvant être prédécoupée en trois parties: une (première) partie basse 5e dont les caractéristiques (forme, angle d'attaque, inclinaison des pales, ...) sont celles d'une pompe centrifuge. Dans une pompe centrifuge le fluide pompé est aspiré axialement puis accéléré radialement, et enfin refoulé tangentiellement. Dans le cas présent, le fluide arrive axialement via la chambre d'admission puis est accéléré radialement grâce à la courbure prononcée du bas du rotor. Cette courbe prononcée est obtenue en utilisant des abaques connus de l'homme du métier tel que les abaques publiés par Sabersky, Acosta et Hauptmann en 1989 (« Fluid Flow: A First Course in Fluid Mechanics », by Rolf H. Sabersky, A. J. Acosta and Edward G. Hauptmann, 3 Sub edition , March 6, 1989, Prentice Hall College Div) ou encore Stepanoff A. (Stepanoff A., "Centrifugal and Axial Flow Pumps", 2nd ed. 1957, New York: Krieger Publishing Company). L'effet centrifuge est rendu de manière optimale par le fait que le carter 6 présente une forme intérieure également conique concave, complémentaire à la forme du rotor 5 sur toute la partie utile.

La seconde partie 5f est de forme mixte selon les abaques. Il s'agit d'une partie intermédiaire suivant la partie centrifuge et présentant une courbure moins prononcée que celle de la partie centrifuge ou alors un plan incliné par rapport à l'axe de rotation.

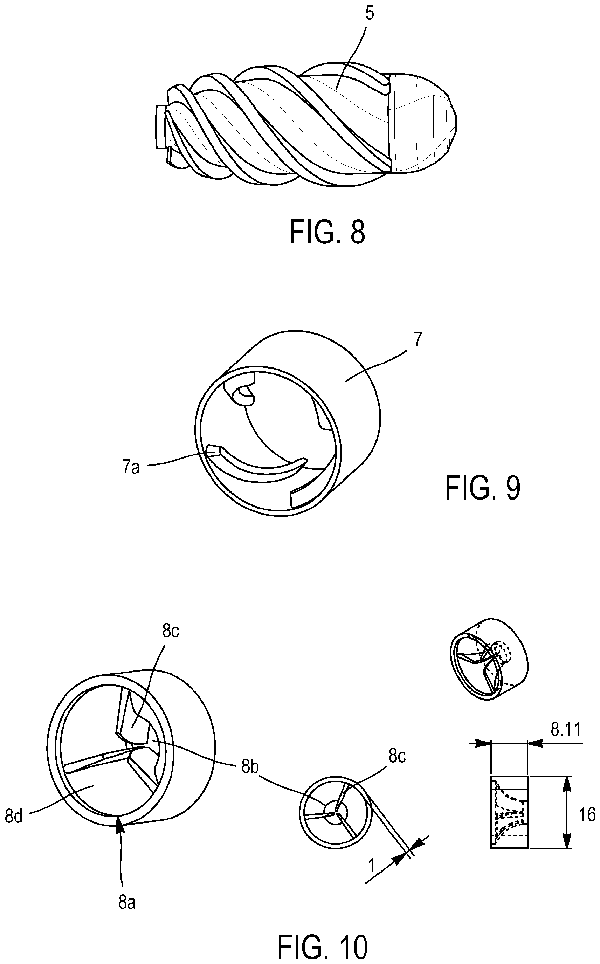

La troisième partie 5g est de forme axiale, la forme extérieure du rotor et la forme intérieure du carter sont sensiblement linéaires et parallèles à l'axe de rotation du rotor. Sur la figure 8 on voit en détail un tel rotor.

Un arbre non représenté est prévu sous forme de tige fixée dans l'axe du rotor. En fonctionnement, l'arbre relie le rotor 5 à un moteur non représenté, l'arbre passant via la chambre d'admission. En rotation, l'arbre tourne sans entrer en contact avec les parois de la chambre d'admission. Pour la fixation, l'arbre et le rotor peuvent être conçus en une pièce unique ou bien l'arbre peut être inséré dans le rotor 5 par l'alésage 5i visible sur la figure 4.

Sur la figure 7 on distingue plus en détail le carter 6 constitué d'un corps principal 6a et d'un corps secondaire 6b. Le corps principal 6a est un cylindre allongé, le corps secondaire étant un cylindre dont le diamètre extérieur est inférieur au diamètre extérieur du corps principal. Le corps secondaire 6b est conformé de façon à s'insérer et y être maintenu fixe dans la partie supérieure 3b de la chambre d'admission. De préférence l'extrémité basse du corps secondaire 6b vient en butée sur la marche 3f, visible sur la figure 5. La forme intérieure du carter 6 est complémentaire à la forme extérieure de la partie utile du rotor sur toute la longueur des pales hélicoïdales. Une partie supérieure du carter présente par contre un diamètre intérieur uniforme de sorte qu'elle constitue un cylindre creux conventionnel de section circulaire. Au niveau de cette partie supérieure, à l'intérieur du carter, on trouve la tête 5h du rotor ainsi que le diffuseur 7 et le redresseur 8.

En fonctionnement, la forme interne complexe du carter permet de réaliser les fonctions centrifuges, mixte et axiale de sorte que le fluide est aspiré dans la pompe sans créer de tourbillon, il est ensuite propulsé vers le haut de la pompe sans cisaillement pour ne pas détruire les globules rouges. Le rotor selon l'invention permet de communiquer l'énergie cinétique au fluide par sa forme particulière. Il modifie donc la vitesse du fluide sans cisaillement et augmente aussi sa pression. Pour se faire, les éléments de sortie de la pompe contribuent à augmenter la pression en présentant un orifice de sortie réduit ainsi que des formes spécifiques.

Sur la figure 9, on voit le diffuseur 7 qui est un cylindre destiné à venir coiffer la tête 5h du rotor. Le diffuseur comporte des pales de guidage orientées 7a dans le sens du refoulement du fluide amené par le rotor. L'orientation des pales de guidage permet de transformer une partie de l'énergie cinétique du fluide en pression qui est une énergie potentielle. L'épaisseur des pales de guidage peut être fixe le long du diffuseur ou bien variable de façon à épouser la forme de la tête du rotor. La hauteur du diffuseur 7 est de préférence sensiblement identique à celle de la tête du rotor. Le tableau ci-dessous présente les propriétés du diffuseur :

| **Diffuseur** | **valeurs** | **Intervalle de Tolérance (intervalle de valeurs possibles)** |
|---|---|---|
| Longueur de la région des pales (*mm)* | **6** | **3-9** |
| Nombre de pales | **4** | **2-5** |
| Espacement maximum (*mm)* | **0.3** | **0.1-0.5** |
| Hauteur de la pale au bord d'attaque (« leading edge ») (*mm)* | **2** | **1-5** |
| Hauteur de la pale au bord de fuite («trailinq edge ») (*mm)* | **3** | **1-5** |
| angle theta au bord d'attaque (*deg)* | **-2.5** | **(-1)-(-40)** |
| angle theta au bord de fuite (*deg)* | **-87.5** | **(-50)-(-150)** |
| angle beta au bord d'attaque (*deg)* | **-75** | **(-15)-(-90)** |
| angle beta au bord de fuite (*deg)* | **-25** | **(-1)-(-40)** |
| Épaisseur de couche normale | **0.95 to 0.525** | **0.25-1.5** |

Sur la figure 10, on voit un redresseur 8 ayant pour rôle de guider le refoulement du fluide en créant un écoulement laminaire de façon à éliminer les turbulences. Il s'agit d'un cylindre ouvert 8a sur sa base pour recevoir le fluide provenant du rotor 5 via le diffuseur 7. Il comporte un orifice 8b de diamètre inférieur par rapport au diamètre de l'ouverture 8a sur sa base.

On distingue une paroi intérieure 8d de forme conique concave depuis l'ouverture 8a sur sa première moitié puis conique convexe sur sa deuxième moitié vers l'orifice 8b. Le fluide est mis sous pression lorsque poussé vers l'orifice de faible diamètre.

On distingue également trois pales de guidage 8c inscrites dans des plans radiaux qui concourent au centre du redresseur. Chaque pale est une lame de largeur plus épaisse du côté de la paroi que du côté du centre du cylindre. La largeur s'affine donc en s'éloignant de la paroi du cylindre.

Dans la configuration décrite, pour chaque pale de guidage, le profil du côté faisant face à l'axe de rotation du cylindre, est courbé, notamment en arc de cercle, de telle sorte que les pales de guidage se rapprochent les unes des autres au niveau de l'orifice et sont plus éloignées du côté de l'ouverture 8a.

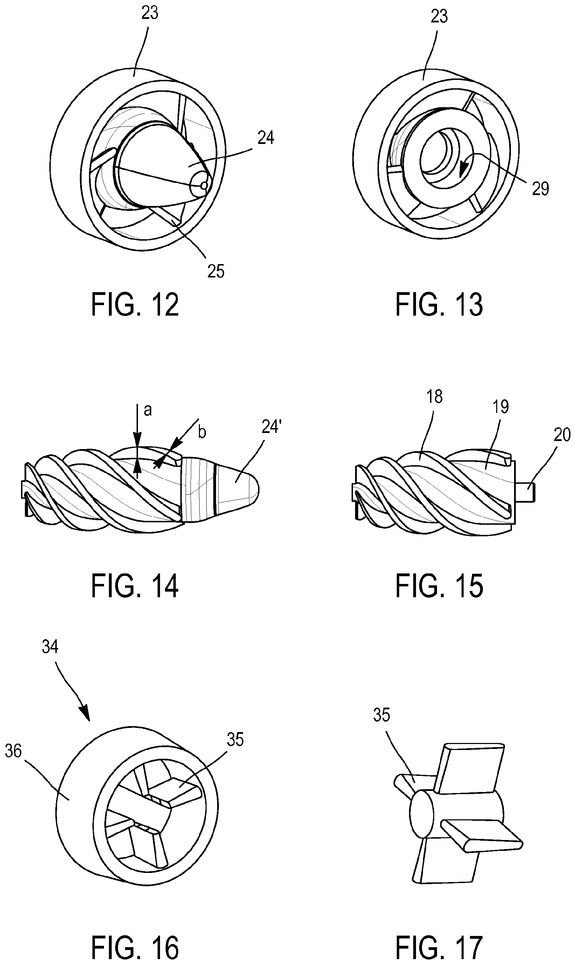

Une variante du redresseur peut être conçue de façon concave avec des pales qui suivent la partie interne concave du redresseur et dont l'épaisseur s'élargit vers l'extrémité 8b de façon linéaire. Cette variante permettant au redresseur d'épouser la forme de la partie 5h sur la figure 4 ou d'une ogive 24' directement fixée au rotor de la figure 14 ou de l'ogive 24 du diffuseur de la figure 12 suivant une autre variante.

En d'autres termes le redresseur s'adapte à la forme de la tête du rotor et/ou du diffuseur utilisés.

Les dimensions pouvant être adoptées sont indiquées dans le tableau ci-dessous :

| **redresseur** | valeurs | Intervalle de Tolérance (intervalle de valeurs possibles) |
|---|---|---|
| Longueur de la région des pales (*mm)* | **8** | **3-9** |
| Nombre de pales | **3** | **2-4** |
| Espacement maximum (*mm)* | **0.3** | **0.1-0.4** |
| Hauteur de la pale au bord d'attaque (« leading edge ») (*mm)* | **3.2** | **1-5** |
| Hauteur de la pale au bord de fuite («trailing edge ») (*mm)* | **3.9** | **1-7** |
| angle theta au bord d'attaque (*deg)* | **-7.65** | **-1-(-30)** |
| angle theta au bord de fuite (*deg)* | **-7.45** | **-1-(-30)** |
| angle beta au bord d'attaque (*deg)* | **-0.2** | **1-(-10)** |
| angle beta au bord de fuite (*deg)* | **0.4** | **1-(-10)** |
| Épaisseur de couche normale | **0.28 to 0.42** | **0.1 to 1** |

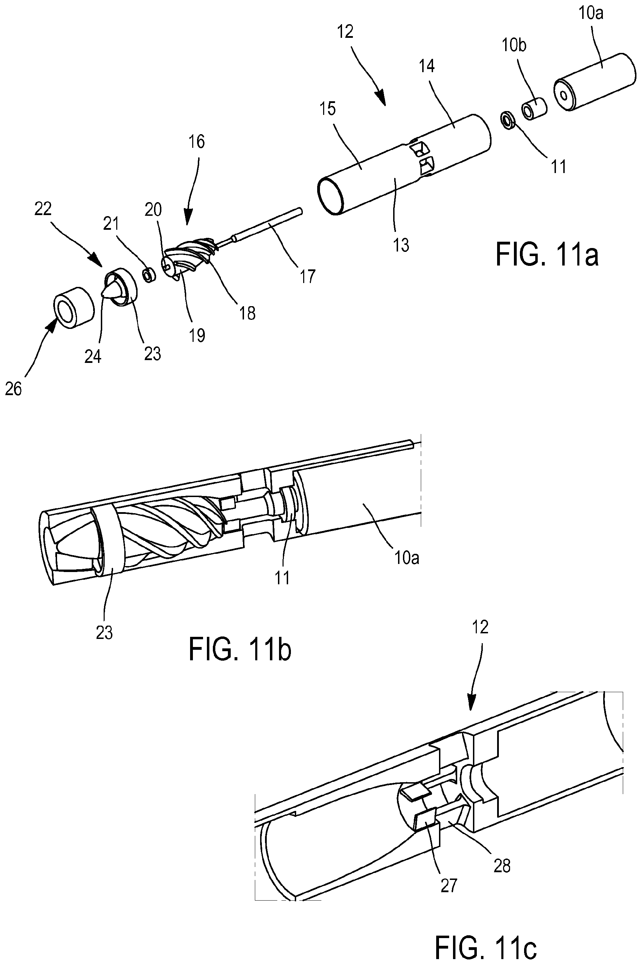

Sur les figures 11a, 11b et 11c est représentée une variante de la pompe selon l'invention. Cette variante est particulièrement adaptée pour diffuser efficacement le sang hors de la pompe. La particularité de cette pompe est son diffuseur 22 disposé à l'extrémité de la zone active du rotor 16 au moyen d'un roulement 21. Cette particularité est illustrée sur la figure 11a ensemble avec d'autres composants de la pompe, ces composants étant différents de ceux décrits sur les autres figures mais on peut naturellement envisager d'utiliser les composants déjà décrits dans la mesure où ils sont compatibles avec la présente particularité.

Plus globalement, on distingue un caisson turbine 12, qui est une pièce de forme cylindrique dotée d'ouvertures latérales 13 pour l'admission de sang. L'homme du métier comprendra aisément que le caisson de turbine regroupe en fait les fonctions du carter, de la chambre d'admission et de l'inducteur décrit précédemment. Ce caisson turbine 12 peut être conçu soit dans une pièce unique qui comprend l'inducteur soit ce caisson peut être créé en deux parties distinctes à laquelle l'inducteur peut être ajouté. En d'autres termes, il peut n'y avoir qu'une seule pièce ou trois pièces distinctes telles que la partie inférieure 14 qui comprend la partie 13, l'inducteur et la partie 15 tels que représenté sur les figures 5, 6 et 7. Les ouvertures latérales 13 permettent de distinguer une partie supérieure 15 du caisson plus haute qu'une partie inférieure 14. La partie supérieure 15 est creuse pour recevoir le rotor 16 ainsi que son arbre d'entrainement 17. Ce dernier est destiné à traverser la partie inférieure 14 par son centre jusqu'à un moteur 10a. En d'autres termes, le moteur 10a maintient l'arbre d'entrainement 17 qui traverse toute la partie inférieure 14 jusqu'au rotor 16 et auquel il est fermement fixé pour l'entrainer en rotation. Le moteur 10a peut être partiellement ou totalement intégré à l'extrémité de la partie inférieure 14, mais il peut également être disposé à l'extérieur. Un joint 11 est prévu à l'intérieur de la partie inférieure 14pour l'étanchéité entre un roulement 10b, le moteur 10a et le sang. Dans tous les cas, ce joint 11 est situé avant la partie 13 et après un roulement 10b qui n'est pas représentée ici et qui sert au guidage de l'arbre d'entraînement 17. Ce roulement 10b peut se situer dans la partie inférieure 14 ou dans le moteur 10a.

La partie supérieure 15 est suffisamment longue pour accueillir en plus du rotor 16, le roulement 21, le diffuseur 22 et le redresseur 26, ces éléments étant connectés en série.

Le rotor 16 est constitué d'un corps central évasé 19 autour duquel sont enroulées quatre pales hélicoïdales 18 sur toute la longueur du corps central. La tête du rotor est coupée à plat, seul un axe 20 dépasse. Sur la figure 15, on voit plus en détail le rotor 16 suivant la variante des figures 11a, 11b et 11c. On note que l'épaisseur des pales 18 n'est pas constante le long du corps central évasé 19. L'axe 20 est une tige mâle dimensionnée pour recevoir le roulement 21 en tant qu'élément femelle.

La bague extérieure du roulement 21 est montée serrée dans le logement 29 figure 13 du diffuseur 22 lui-même fixé à l'intérieur de la partie 15 du caisson 12. L'axe 20 du rotor 16 est monté glissant dans la bague intérieure du roulement 21 permettant ainsi le guidage en rotation du rotor au même titre que le roulement 10b du moteur 10a.

Le redresseur 26 est fixé dans la partie supérieure 15 du caisson 12, au même titre que le diffuseur 22. Il s'agit d'un cylindre creux doté de pales droites disposées radialement. Lorsque la pompe est complètement montée, le redresseur 26 affleure ou est en retrait (à l'intérieur) de l'extrémité de la partie supérieure 15. L'ensemble avec le moteur fait une longueur de moins de 100mm.

En fonctionnement, le moteur 10a de type « brushless » fait tourner l'ensemble 16 qui est composé des pièces 17 à 20. Le sang pénètre par les ouvertures 13, passe ensuite par un inducteur (non représenté) disposé à l'intérieur du caisson 12. Cet inducteur peut être sous la forme de plusieurs pales de guidages droites radialement fixées à la paroi interne du caisson 12, « au pied » de la partie supérieure 15. Le sang est aspiré par le rotor 16 et passe tout autour de l'arbre d'entrainement 17 sous forme de flux linéaire. Le sang est ensuite entrainé par le rotor en tournant jusque dans le diffuseur qui porte des pales vrillées dans le sens opposé au sens des pales hélicoïdales. Le flux de sang cesse alors de tourner et est ensuite redressé en passant par le redresseur 26 qui par son orifice de sortie crée un flux laminaire de forte pression. La pompe est prévue pour fonctionner en immersion selon une fréquence allant de 500 à 10000 tr/min.

Sur les figures 11b et 11c, on distingue l'intérieur du caisson turbine 12 conformé de façon à accueillir les différents composants. La partie supérieure 15 comporte en intérieur une forme évasée venant épouser, sans toucher, la forme extérieure du rotor 16. Les pales 27 sont directement réalisées sur la paroi interne du caisson, formant ainsi un inducteur à l'extrémité amont de la partie supérieure 15. Les ouvertures 13 sont directement réalisées dans le caisson de sorte que subsistent uniquement des lames 28 inscrites dans des plans concurrents à l'axe de révolution du caisson. L'arbre d'entrainement 17 est prévu le long de l'axe de révolution du caisson comme on peut le voir sur la figure 11b. Le fluide est destiné à pénétrer radialement par les ouvertures 13 et à être entrainé tout autour de l'arbre d'entrainement 17 en direction du rotor, entre les pales hélicoïdales, jusqu'à travers le diffuseur 22. Les pales du redresseur peuvent également être directement réalisées sur la paroi interne à l'extrémité aval de la partie supérieure 15.

Le joint 11 sur la figure 11b empêche le fluide de descendre vers le moteur 10a. Dans l'exemple illustré sur la figure 11b, le roulement 10b est intégré dans le moteur 10a qui lui-même pénètre dans la partie inférieure 14. Les figures 11b et 11c sont des variantes pour lesquelles l'inducteur et le redresseur sont directement réalisés dans le caisson.

Les dimensions du rotor peuvent être comme illustrées dans le tableau ci-dessous.

| **Rotor** | valeurs | **Plage de valeurs possibles** |
|---|---|---|
| Longueur de la région avec pales hélicoïdales (mm) | **25** | **10-35** |
| nombre de pales hélicoïdales (mm) | **4** | **2-5** |
| gap entre rotor et carter (mm) | **0.3** | **0.1-0.4** |
| Hauteur de la pale au bord d'attaque (« leading edge ») du rotor (mm) | **2.9** | **1-5** |
| Hauteur de la pale au bord de fuite («trailing edge ») du rotor (mm) | **2** | **1-5** |
| Angle Beta au bord d'attaque du rotor (deg) | **47.5** | **10-90** |
| Angle Beta au bord de fuite du rotor (deg) | **22** | **10-90** |
| Angle theta au bord d'attaque du rotor (deg) | **0.2** | **0-50** |
| Angle theta au bord de fuite du rotor (deg) | **312** | **100-360** |
| Epaisseur de la pale hélicoïdale (mm) | **0.2-1.4** | **0.1-2** |

Les angles d'attaque et de sortie du sang dans le rotor sont tels que le sang est propulsé à l'entrée par une force centrifuge, et est relâché en sortie par une force axiale, la zone centrale du rotor étant semblable à une force mixte. On note que la hauteur de la pale hélicoïdale (son épaisseur selon la direction radiale) peut varier, par exemple décroître entre le bas et le haut du rotor. Par ailleurs, l'épaisseur latérale de la pale hélicoïdale peut également varier, par exemple croître entre le bas et le haut du rotor. La hauteur « a » et l'épaisseur latérale « b » sont schématiquement représentées sur la figure 14. Les mêmes dimensions peuvent être appliquées pour les différents modes de réalisations.

Sur la figure 12 est illustré plus en détail un diffuseur 22 selon l'invention. Il est composé d'un cylindre 23 relié à une ogive 24 par des pales vrillées 25. Sur la figure 13 on distingue l'arrière du diffuseur 22. On note que l'ogive 24 présente sur sa face arrière le logement 29 prévu pour recevoir le roulement 21 et l'axe 20.

Sur la figure 14, on voit une autre variante pour laquelle l'ogive 24' est intégrée et pivote avec le rotor. Dans ce cas, le diffuseur est constitué d'un cylindre comme le cylindre 23 mais avec des pales vrillées uniquement fixées à ce cylindres et permettant le passage de la tête d'ogive avec une épaisseur de marge pour éviter tout frottement.

Sur les figures 16 et 17, on distingue une représentation schématique d'un inducteur individuel 34 sur la figure 16 et une représentation schématique uniquement des pales 35 de cet inducteur sur la figure 17. Cet inducteur peut être conçu individuellement. Il sera alors à fixer fermement dans une chambre d'admission. Il peut également être directement réalisé dans une chambre d'admission ou dans un caisson turbine ; dans ce cas ce sont les pales qui sont réalisées sur la paroi interne de la chambre d'admission ou du caisson turbine. La zone centrale de l'inducteur est laissée libre pour le passage du fluide. Sur la figure 17, la zone centrale de l'inducteur représente le flux du fluide.

De façon générale et pour l'ensemble des modes de réalisation, les pales de l'inducteur selon l'invention sont plus épaisses en amont qu'en aval dans le sens de déplacement du fluide. La progression de l'épaisseur peut être linéaire, mais de préférence discontinue : une progression linéaire jusqu'à atteindre une certaine épaisseur, puis l'épaisseur reste constante sur le reste de la longueur de la pale. Par ailleurs, les pales peuvent également être plus épaisses à l'endroit de liaison avec le cylindre 36 qui les porte qu'à l'extrémité centrale. On prévoit également un angle entre la section radiale de chaque pale et le rayon du cylindre 36 portant les pales. Les données numériques sont indiquées sur le tableau ci-dessous :

| **Inducteur** | **valeurs** | **Intervalle de Tolérance (intervalle de valeurs possibles)** |
|---|---|---|
| Longueur de la région de l'inducteur (*mm)* | **4.5** | **3-8** |
| Nombre de pales de l'inducteur | **4** | **3-6** |
| Espacement maximum (*mm)* | **0.28** | **0.1-0.3** |
| Longueur de la région des pales de l'inducteur (*mm)* | **2.9** | **1-5** |
| angle θ₁ au bord d'attaque (deg) | **74.41** | **30-90** |
| angle θ₂ au bord de fuite (deg) | **73.7** | **30-90** |
| angle β₁ au bord d'attaque (deg) | **0** | **-10-30** |
| angle β₂ au bord de fuite (deg) | **1.2** | **-10-30** |
| Épaisseur de couche normale (*mm)* | **035-0.95** | **0.2-1** |

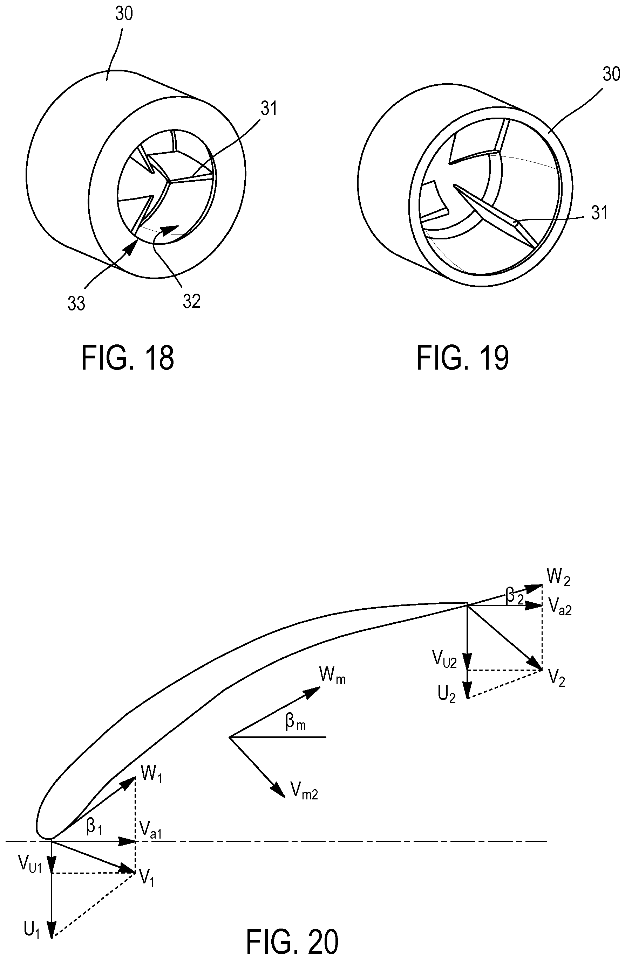

La figure 20 est une vue en coupe d'une pale sur laquelle sont illustrés différents paramètres de conception. Les définitions des différents paramètres sont illustrées par la figure ci-dessous :
On distingue une pale 37 sur laquelle sont définis un bord d'attaque ("leading edge (inlet)") et un bord de fuite ("trailing edge (outlet)"). Les paramètres suivants permettent de caractériser une pale lors de la conception :
Pour la zone d'entrée :
   U₁ = vitesse de la pale,
   V₁ =vitesse du fluide après contact avec la pale (vitesse de sortie/vitesse finale)
   W₁ = vitesse du fluide avant contact avec la pale (vitesse d'entrée/vitesse initiale)
   V_{U1} = projection du vecteur vitesse V1 sur l'axe U₁
   Vₐ₁ = Projection de V₁ et W₁ sur l'axe défini par l'axe de la turbine
   β₁ = angle entre le vecteur W₁ et l'axe de la turbine
   θ₁ = angle entre le vecteur V₁ et l'axe de la turbine
Pour la zone de sortie :
   U₂ = vitesse de la pale,
   V₂ =vitesse du fluide après contact avec la pale (vitesse de sortie/vitesse finale)
   W₂ = vitesse du fluide avant contact avec la pale (vitesse d'entrée/vitesse initiale)
   V_{U2} = projection du vecteur vitesse V₂ sur l'axe U₂
   Vₐ₂ = Projection de V₂ et W₂ sur l'axe défini par l'axe de la turbine
   β₂ = angle entre le vecteur W₂ et l'axe de la turbine
   θ₂ = angle entre le vecteur V₂ et l'axe de la turbine
Pour la zone centrale :
   Vₘ =vitesse du fluide après contact avec la pale (vitesse de sortie/vitesse finale)
   Wₘ = vitesse du fluide avant contact avec la pale (vitesse d'entrée/vitesse initiale)
   βₘ = angle entre le vecteur Wₘ et l'axe de la turbine
   θₘ = angle entre le vecteur Vₘ et l'axe de la turbine

Les ensembles U, V et W constituent des triangles de vitesse de flux et servent de référentiel pour la définition des vecteurs vitesses et des angles βₘ et θₘ, où m est égale à 1 pour le repère dont l'origine a été déplacé au bord d'attaque, et 2 pour le repère dont l'origine a été déplacé au bord de fuite. Le trait en pointillé représente la direction axiale.

Les figures 18 et 19 illustrent une vue de face et une vue arrière d'un redresseur selon l'invention. Ce redresseur peut être réalisé de façon individuelle puis fixé dans le carter de la pompe ou bien directement réalisé sur la paroi interne du carter, c'est-à-dire une paroi conformée identiquement à la paroi interne du cylindre 30 du redresseur et des pales conçues directement sur cette paroi. Les pales 31 sont plus épaisses en amont qu'en aval dans le sens de circulation du fluide. La progression de l'épaisseur peut être linéaire. Sur la figure 18 est illustré le côté aval du redresseur, c'est-à-dire l'endroit par lequel sort le fluide. Sur la figure 19 est illustré le côté amont du redresseur, l'entrée du fluide. Sur ce dernier côté, les pales 31 laissent un espace centrale plus grand que du côté aval, les pales et la paroi interne 32 du cylindre 30 portant les pales étant conçus de façon à guider le fluide vers l'orifice de sortie 33 qui est plus étroit que l'entrée du redresseur.

Bien sûr, l'invention n'est pas limitée aux exemples qui viennent d'être décrits et de nombreux aménagements peuvent être apportés à ces exemples sans sortir du cadre de l'invention.

La pompe selon l'invention peut facilement être implantée dans un cœur par sa petite dimension du fait de son design particulier permettant une pression élevée tout en conservant la qualité du sang.

La pompe selon l'invention consomme peu du fait qu'elle fonctionne selon le rythme cardiaque physiologique : un flux oscillant.

La pompe selon l'invention fonctionne en propulsant : le rythme est pulsé.

La pompe selon l'invention est avantageusement prévue pour fonctionner à la verticale, le rotor étant disposé à la verticale, le fluide pénètre via l'inducteur, traverse le rotor puis ressort par le haut via le diffuseur et le redresseur. La plupart des pompes de l'art antérieur fonctionne à l'horizontale. C'est la capacité d'admission et de refoulement qui permet le fonctionnement à la verticale de la pompe selon l'invention. Une telle pompe, placée dans un ventricule gauche par exemple, présente l'avantage d'avoir une entrée et une sortie directement dans ce ventricule. Ceci permet d'éviter la présence d'un tube d'entrée et/ou de sortie comme il existe sur les autres dispositifs de l'art antérieur.

## Revendications

1. Pompe (1) destinée à être immergée dans un fluide comprenant :
- un inducteur (4) doté de pales de guidage (4a) pour rendre l'écoulement du fluide linéaire ;
**caractérisée en ce qu'**elle comprend en outre :
- un rotor (5, 16) comprenant un corps central (19) de forme évasée, ce rotor (5, 16) étant destiné à créer de l'énergie cinétique et étant disposé en aval de l'inducteur (4) par rapport au sens de circulation du fluide ;
- un arbre (17) d'entrainement et de maintien du rotor (5, 16), cet arbre étant fixé sur l'extrémité amont du rotor (5, 16) et traversant la chambre d'admission (3) dans sa zone axiale ;
- au moins une pale hélicoïdale (18) réalisée autour dudit corps central ; cette pale hélicoïdale (18) ayant un profil extérieur évasé et comporte des spires à pas d'enroulement croissant et tendant vers l'infini, et le volume intérieur du carter (6) étant complémentaire à la forme évasée de ladite au moins une pale hélicoïdale (18),
- un carter (6) autour du rotor (5, 16) ;
- un diffuseur (7, 22) doté de pales pour rendre l'écoulement de fluide linéaire et augmenter la pression du fluide ; ce diffuseur (7, 22) étant disposé en aval du rotor (5, 16) de façon à évacuer le fluide depuis le rotor (5, 16) vers l'extérieur en transformant l'énergie cinétique créée par le rotor (5, 16) en énergie potentielle ; et
- un redresseur (8, 26) doté de pales (8c) et d'un orifice (8b) en sortie de diamètre inférieur au diamètre d'entrée du redresseur (8, 26), les pales (8c) dirigeant le fluide, en provenance du diffuseur (7, 22), vers l'orifice de façon à augmenter la vitesse et donner au fluide un profil prédéterminé en sortie de l'orifice.

2. Pompe selon la revendication 1, **caractérisée en ce qu'**elle comprend en outre une chambre d'admission (3) pourvue d'ouvertures latérales pour que le fluide puisse pénétrer radialement puis s'engager dans l'axe vers l'inducteur (4).

3. Pompe selon la revendication 2, **caractérisée en ce que** la chambre d'admission (3) est de forme cylindrique comportant sur sa partie supérieure en aval desdites ouvertures, un réceptacle pour accueillir l'inducteur (4).

4. Pompe selon l'une quelconque des revendications précédentes, **caractérisée en ce qu'**elle comporte en tout quatre pales hélicoïdales (18) identiques réparties de façon homogène autour du corps central.

5. Pompe selon l'une quelconque des revendications précédentes, **caractérisée en ce que** le corps central du rotor (5, 16) est de forme oblongue.

6. Pompe selon l'une quelconque des revendications précédentes, **caractérisée en ce que** la tête du corps central est arrondie et dépourvue de pale.

7. Pompe selon l'une quelconque des revendications précédentes, **caractérisée en ce que** le diffuseur (7, 22) est un cylindre creux doté de pales de guidage droites réparties dans sa paroi interne et s'étendant de la périphérie vers le centre.

8. Pompe selon l'une quelconque des revendications 1 à 6, **caractérisée en ce que** le diffuseur (7, 22) est un cylindre creux ayant, réparties dans sa paroi interne, des pales de guidage vrillées (7a) dans un sens inverse au sens d'enroulement de la pale hélicoïdale (18) autour du corps central.

9. Pompe selon la revendication 7 ou 8, **caractérisée en ce que** lorsque le corps central comporte une tête arrondie dépourvue de pales, le diffuseur (7, 22) coiffe cette tête arrondie, et chaque pale de guidage du diffuseur (7, 22) présente une forme complémentaire à une partie de la tête arrondie faisant face.

10. Pompe selon l'une quelconque des revendications précédentes, **caractérisée en ce que** le redresseur (8, 26) est disposé en aval du diffuseur (7, 22) selon le sens de circulation du fluide ; la paroi interne du redresseur (8, 26) étant de forme conique ; des pales de guidage (8c) droites étant disposées sur cette paroi interne.

11. Pompe selon l'une quelconque des revendications 1 à 4, **caractérisée en ce que** le diffuseur (7, 22) comporte une partie centrale en forme d'ogive pointue vers l'aval, un cylindre autour de la base de l'ogive, des pales de guidage (7a) reliant ce cylindre à la base de l'ogive ; ce diffuseur (7, 22) étant destiné à venir en prise avec l'extrémité aval du rotor (5, 16) via un roulement permettant de garder le diffuseur (7, 22) fixe par rapport au rotor (5, 16).

12. Pompe selon la revendication 11, **caractérisée en ce que** les pales de guidage du diffuseur (7, 22) sont vrillées dans un sens inverse au sens d'enroulement de la pale hélicoïdale (18) autour du corps central.

13. Pompe selon la revendication 11 ou 12, **caractérisée en ce que** le redresseur (8, 26) est disposé en aval du diffuseur (7, 22) selon le sens de circulation du fluide ; des pales de guidage sont droites et sont conçues de façon à laisser pénétrer la tête de l'ogive dans la partie centrale du redresseur (8, 26).

14. Pompe selon l'une quelconque des revendications précédentes, **caractérisée en ce que** l'ensemble rotor (5, 16) et pale hélicoïdale (18) présente un profil de type centrifuge du côté amont, de type mixte dans la partie centrale, et de type axial du côté aval.

15. Pompe selon l'une quelconque des revendications précédentes, **caractérisée en ce que** la partie du côté amont de l'ensemble corps central et pale hélicoïdale (18) est dimensionnée pour une vitesse spécifique comprise entre 0 et 1.2.

16. Pompe selon l'une quelconque des revendications précédentes, **caractérisée en ce que** la partie centrale de l'ensemble corps central et pale hélicoïdale (18) est dimensionnée pour une vitesse spécifique comprise entre 1 et 2.2.

17. Pompe selon l'une quelconque des revendications précédentes, **caractérisée en ce que** la partie centrale de l'ensemble corps central et pale hélicoïdale (18) est dimensionnée pour une vitesse spécifique supérieure à 2.2.

18. Pompe selon l'une quelconque des revendications précédentes, **caractérisée en ce que** la chambre d'admission (3), l'inducteur, le carter (6) et le diffuseur (7, 22) sont conçus en une seule pièce (12) ou sont fermement fixés les uns aux autres sans mouvements relatifs, le rotor (5, 16) étant maintenu mobile en rotation dans le carter (6).

19. Pompe selon l'une quelconque des revendications précédentes, **caractérisée en ce que** ladite au moins une pale hélicoïdale (18) présente une hauteur radiale identique sur toute sa longueur.

20. Pompe selon l'une quelconque des revendications précédentes, **caractérisée en ce que** ladite au moins une pale hélicoïdale (18) présente une hauteur radiale variable sur toute sa longueur.

## Patentansprüche

1. Pumpe (1), die ausgebildet ist zum Eintauchen in eine Flüssigkeit, mit
- einem Induktor (4), der mit Leitflügeln (4a) versehen ist, um die Strömung des Fluides zu linearisieren;
**dadurch gekennzeichnet, dass** die Pumpe des Weiteren das Folgende aufweist:
- einen Rotor (5, 16) mit einem aufgeweiteten zentralen Körper (19), wobei der Rotor (5, 16) dazu eingerichtet ist, kinetische Energie zu erzeugen und bezüglich der Strömungsrichtung des Fluides dem Induktor (4) nachgelagert angeordnet ist;
- eine Antriebs- und Stützwelle (17) des Rotors (5, 16), wobei die Welle an der vorgelagerten Extremität des Rotors (5, 16) festgelegt ist und die Einlasskammer (3) in ihrer axialen Zone durchquert;
- zumindest einen helikalen Flügel (18), der um den zentralen Körper herum angeordnet ist; wobei der helikale Flügel (18) ein äußeres, aufgeweitetes Profil und Windungen hat, deren Ganghöhe zunimmt und gegen unendlich strebt, und wobei das Innenvolumen des Gehäuses (6) zu der aufgeweiteten Form des zumindest einen helikalen Flügels (18) komplementär ist,
- ein Gehäuse (6), das um den Rotor (5, 16) angeordnet ist;
- ein Diffusor (7, 22), der mit Flügeln versehen ist, um die Strömung des Fluides zu linearisieren und den Druck des Fluides zu erhöhen; wobei der Diffusor (7, 22) dem Rotor (5, 16) nachgelagert ist, um das Fluid von dem Rotor (5, 16) nach außen abzuführen, indem die durch den Rotor (5, 16) erzeugte kinetische Energie in potentielle Energie umgewandelt wird; und
- ein Gleichrichter (8, 26), der mit Flügeln (8c) und einer Öffnung (8b) am Ausgang mit kleinerem Durchmesser als der Durchmesser des Eingangs des Gleichrichters (8, 26) versehen ist, wobei die Flügel (8c) das Fluid, das von dem Diffusor (7, 22) kommt, zu der Öffnung leiten, um die Geschwindigkeit zu erhöhen und dem Fluid bei Austreten aus der Öffnung ein vorbestimmtes Profil zu verleihen.

2. Pumpe nach Anspruch 1, **dadurch gekennzeichnet, dass** sie weiterhin eine Einlasskammer (3) mit lateralen Öffnungen aufweist, damit das Fluid lateral eindringen und sich dann in der Achse in Richtung des Induktors (4) weiterbewegen kann.

3. Pumpe nach Anspruch 2, **dadurch gekennzeichnet, dass** die Einlasskammer (3) zylinderförmig ist und auf ihrer Oberseite, die den Öffnungen vorgelagert ist, einen Behälter aufweist, um den Induktor (4) aufzunehmen.

4. Pumpe nach eine der vorstehenden Ansprüche, **dadurch gekennzeichnet, dass** sie insgesamt vier identische helikale Flügel (18) aufweist, die gleichartig um den zentralen Körper verteilt sind.

5. Pumpe nach einem der vorstehenden Ansprüche, **dadurch gekennzeichnet, dass** der zentrale Körper des Rotors (5, 16) eine längliche Form aufweist.

6. Pumpe nach einem der vorstehenden Ansprüche, **dadurch gekennzeichnet, dass** der Kopf des zentralen Körpers abgerundet ist und keine Flügel aufweist.

7. Pumpe nach einem der vorstehenden Ansprüche, **dadurch gekennzeichnet dass** der Diffusor (7, 22) ein Hohlzylinder ist, der mit geraden Leitflügeln versehen ist, die in dessen Innenwandung verteilt sind und sich von dem Außenrand zur Mitte hin erstrecken.

8. Pumpe nach einem der Ansprüche 1 bis 6, **dadurch gekennzeichnet, dass** der Diffusor (7, 22) ein Hohlzyinder ist, in dessen Innenwand Leitflügel (7a) verteilt sind, die in einem der Wicklungsrichtung des helikalen Flügels (18) um den zentralen Körper herum entgegengesetzten Drehsinn verdreht sind.

9. Pumpe nach Anspruch 7 oder 8, **dadurch gekennzeichnet, dass**, wenn der zentrale Körper einen abgerundeten Kopf ohne Flügel aufweist, der Diffusor (7, 22) diesen abgerundeten Kopf bedeckt, und dass jeder Leitflügel des Diffusors (7, 22) eine dem ihm zugewandten Teil des abgerundeten Kopfs komplementäre Form hat.

10. Pumpe nach einem der vorstehenden Ansprüche, **dadurch gekennzeichnet, dass** der Gleichrichter (8, 26) dem Diffusor (7, 22) bezüglich der Strömungsrichtung des Fluides vorgelagert ist; wobei die Innenwand des Gleichrichters (8, 26) eine konische Form hat und auf dieser Innenwand gerade Leitflügel (8c) angeordnet sind.

11. Pumpe nach einem der Ansprüche 1 bis 4, **dadurch gekennzeichnet, dass** der Diffusor (7, 22) einen zentralen, spitzbogenförmigen Teil aufweist, dessen Spitze in Strömungsrichtung weist, sowie einen Zylinder, der um die Basis des Spitzbogens herum verläuft, sowie Leitflügel (7a), die den Zylinder mit der Basis des Spitzbogens verbinden, aufweist; wobei der Diffusor (7, 22) eingerichtet ist, um mit der in Strömungsrichtung nachgelagerten Extremität des Rotors (5, 16) mittels einer Laufrolle in Eingriff zu treten, die es ermöglicht, den Diffusor (7, 22) bezüglich des Rotors (5, 16) stationär zu halten.

12. Pumpe nach Anspruch 11, **dadurch gekennzeichnet, dass** die Leitflügel des Diffusors (7, 22) in einem der Wicklungsrichtung des helikalen Flügels (18) um den zentralen Körper herum entgegengesetzten Drehsinn verdreht sind.

13. Pumpe nach Anspruch 11 oder 12, **dadurch gekennzeichnet, dass** der Gleichrichter (8, 26) dem Diffusor (7, 22) bezüglich der Strömungsrichtung des Fluides nachgelagert ist; wobei die Leitflügel gerade sind und eingerichtet sind, um die Spitze des Spitzbogens in den zentralen Teil des Gleichrichters (8, 26) eintreten zu lassen.

14. Pumpe nach einem der vorstehenden Ansprüche, **dadurch gekennzeichnet, dass** die Gesamtheit aus Rotor (5, 16) und helikalem Flügel (18) auf der in Strömungsrichtung vorgelagerten Seite zentrifugale Eigenschaften, im zentralen Teil gemischte Eigenschaften und auf der in Strömungsrichtung nachgelagerten Seite axiale Eigenschaften aufweist.

15. Pumpe nach einem der vorstehenden Ansprüche, **dadurch gekennzeichnet, dass** der Teil der in Strömungsrichtung vorgelagerten Seite des Verbunds aus zentralem Körper und helikalem Flügel (18) für eine spezifische Geschwindigkeit zwischen 0 und 1,2 dimensioniert ist.

16. Pumpe nach einem der vorstehenden Ansprüche, **dadurch gekennzeichnet, dass** der zentrale Teil des Verbunds aus zentralem Körper und helikalem Flügel (18) für eine spezifische Geschwindigkeit zwischen 1 und 2,2 dimensioniert ist.

17. Pumpe nach einem der vorstehenden Ansprüche, **dadurch gekennzeichnet, dass** der zentrale Teil des Verbunds aus zentralem Körper und helikalem Flügel (18) für eine spezifische Geschwindigkeit größer 2,2 dimensioniert ist.

18. Pumpe nach einem der vorstehenden Ansprüche, **dadurch gekennzeichnet, dass** die Einlasskammer (3), der Induktor, das Gehäuse (6) und der Diffusor (7, 22) einstückig als nur ein Bauteil (12) ausgebildet sind oder fest aneinander ohne relative Bewegungen befestigt sind, wobei der Rotor (5, 16) in dem Gehäuse (6) beweglich in Rotation gehalten wird.

19. Pumpe nach einem der vorstehenden Ansprüche, **dadurch gekennzeichnet, dass** der zumindest eine helikale Flügel (18) eine auf seiner gesamten Länge identische radiale Höhe aufweist.

20. Pumpe nach einem der vorstehenden Ansprüche, **dadurch gekennzeichnet, dass** der zumindest eine helikale Flügel (18) eine auf seiner gesamten Länge variable radiale Höhe aufweist.

## Claims

1. Pump (1) intended to be submerged in a fluid comprising:
- an inducer (4) equipped with guide blades (4a) to make the flow of the fluid linear;
**characterized in that** it also comprises:
- a rotor (5, 16) comprising a central body (19) having a flared shape, this rotor (5, 16) being intended to create kinetic energy and being arranged downstream of the inducer (4) with respect to the direction of flow of the fluid;
- a shaft (17) for driving and supporting the rotor (5, 16), this shaft being fixed on the upstream end of the rotor (5, 16) and passing through the inlet chamber (3) in its axial area;
- at least one helical blade (18) produced around said central body; this helical blade (18) having a flared external profile and comprising turns with an increasing winding pitch that tends towards infinity, and the internal volume of the casing (6) being complementary to the flared shape of said at least one helical blade (18),
- a casing (6) around the rotor (5, 16);
- a diffuser (7, 22) equipped with blades to make the flow of fluid linear and increase the pressure of the fluid; this diffuser (7, 22) being arranged downstream of the rotor (5, 16) so as to evacuate the fluid outwards from the rotor (5, 16), converting the kinetic energy created by the rotor (5, 16) into potential energy; and
- a straightener (8, 26) equipped with blades (8c) and an outlet orifice (8b) having a diameter smaller than the inlet diameter of the straightener (8, 26), the blades (8c) directing the fluid originating from the diffuser (7, 22) to the orifice so as to increase the speed and give the fluid a predefined profile when leaving the orifice.

2. Pump according to claim 1, **characterized in that** it also comprises an inlet chamber (3) provided with side openings so that the fluid can enter radially then engage axially towards the inducer (4).

3. Pump according to claim 2, **characterized in that** the inlet chamber (3) is cylindrical in shape comprising, on its upper part downstream of said openings, a receptacle for housing the inducer (4).

4. Pump according to any one of the preceding claims, **characterized in that** it comprises in total four identical helical blades (18) uniformly distributed around the central body.

5. Pump according to any one of the preceding claims, **characterized in that** the central body of the rotor (5, 16) is oblong in shape.

6. Pump according to any one of the preceding claims, **characterized in that** the head of the central body is rounded and not provided with a blade.

7. Pump according to any one of the preceding claims, **characterized in that** the diffuser (7, 22) is a hollow cylinder equipped with straight guide blades distributed in its internal wall and extending from the periphery to the center.

8. Pump according to any one of claims 1 to 6, **characterized in that** the diffuser (7, 22) is a hollow cylinder having, distributed in its internal wall, guide blades (7a) twisted in a direction opposite to the direction of winding of the helical blade (18) around the central body.

9. Pump according to claim 7 or 8, **characterized in that** when the central body comprises a rounded head not provided with a blade, the diffuser (7, 22) caps this rounded head, and each guide blade of the diffuser (7, 22) has a shape that is complementary to a facing part of the rounded head.

10. Pump according to any one of the preceding claims, **characterized in that** the straightener (8, 26) is arranged downstream of the diffuser (7, 22) in the direction of flow of the fluid; the internal wall of the straightener (8, 26) being conical in shape; straight guide blades (8c) being arranged on this internal wall.

11. Pump according to any one of claims 1 to 4, **characterized in that** the diffuser (7, 22) comprises a central part in the shape of an ogive that is pointed in the downstream direction, a cylinder around the base of the ogive, guide blades (7a) connecting this cylinder to the base of the ogive; this diffuser (7, 22) being intended to engage with the downstream end of the rotor (5, 16) via a bearing making it possible to keep the diffuser (7, 22) stationary with respect to the rotor (5, 16).

12. Pump according to claim 11, **characterized in that** the guide blades of the diffuser (7, 22) are twisted in a direction opposite to the direction of winding of the helical blade (18) around the central body.

13. Pump according to claim 11 or 12, **characterized in that** the straightener (8, 26) is arranged downstream of the diffuser (7, 22) in the direction of flow of the fluid; guide blades are straight and are designed so as to allow the head of the ogive to enter the central part of the straightener (8, 26).

14. Pump according to any one of the preceding claims, **characterized in that** the rotor (5, 16) and helical blade (18) assembly has a profile of centrifugal type on the upstream side, of mixed-flow type in the central part and of axial type on the downstream side.

15. Pump according to any one of the preceding claims, **characterized in that** the upstream-side part of the central body and helical blade (18) assembly is dimensioned for a specific speed comprised between 0 and 1.2.

16. Pump according to any one of the preceding claims, **characterized in that** the central part of the central body and helical blade (18) assembly is dimensioned for a specific speed comprised between 1 and 2.2.

17. Pump according to any one of the preceding claims, **characterized in that** the central part of the central body and helical blade (18) assembly is dimensioned for a specific speed greater than 2.2.

18. Pump according to any one of the preceding claims, **characterized in that** the inlet chamber (3), the inducer, the casing (6) and the diffuser (7, 22) are designed in a single piece (12) or are firmly fixed to one another without relative movements, the rotor (5, 16) being held rotationally mobile in the casing (6).

19. Pump according to any one of the preceding claims, **characterized in that** said at least one helical blade (18) has a radial height that is identical over its entire length.

20. Pump according to any one of the preceding claims, **characterized in that** said at least one helical blade (18) has a radial height that is variable over its entire length.
